# EUROPÄISCHE PATENTSCHRIFT

(11) **EP 0 711 267 B1**
(45) Veröffentlichungstag und Bekanntmachung des Hinweises auf die Patenterteilung: **15.10.1997**
(21) Anmeldenummer: 94923711.9
(22) Anmeldetag: 07.07.1994
(51) Int. Cl.: C07C 31/20, C07C 29/00

(54) **VERFAHREN ZUR HERSTELLUNG VON 1,4-BUTANDIOL**
PROCESS FOR PRODUCING 1,4 BUTANE DIOL
PROCEDE DE FABRICATION DE 1,4-BUTANEDIOL

(30) Priorität: 31.07.1993 DE 4325753
(43) Veröffentlichungstag der Anmeldung: 15.05.1996
(73) Patentinhaber: BASF AKTIENGESELLSCHAFT, 67056 Ludwigshafen (DE)
(72) Erfinder: PINKOS, Rolf, D-67098 Bad Dürkheim (DE); FISCHER, Rolf, Harthmuth, D-69121 Heidelberg (DE); BREITSCHEIDEL, Boris, D-67117 Limburgerhof (DE); POLANEK, Peter, D-69469 Weinheim (DE)
(86) Internationale Anmeldenummer: EP9402232
(87) Internationale Veröffentlichungsnummer: WO9504023

(56) Entgegenhaltungen:
- EP-A- 0 340 970
- EP-A- 0 343 841
- EP-A- 0 388 868
- WO-A-92/20667
- BE-A- 674 652
- DE-A- 2 519 817
- DE-A- 2 825 586
- US-A- 4 475 004
- US-A- 4 476 332

## Beschreibung

Die vorliegende Erfindung betrifft ein Verfahren zur Herstellung von 1,4-Butandiol.

In US-A-4 209 651 wird erwähnt, daß 2,5-Dihydrofuran zur direkten Herstellung von 1,4-Butandiol verwendet werden kann. Es wird in diesem Patent aber nicht angegeben, durch welche Maßnahmen dies zu bewerkstelligen wäre.

WO 92/20 667 lehrt hingegen ein Verfahren zur Herstellung von 1,4-Butandiol aus 2,5-Dihydrofuran, in dem zunächst in einer ersten Stufe 2,5-Dihydrofuran in Gegenwart homogen im Reaktionsmedium gelöster Rhodium- oder Ruthenium-Phosphin-Komplexe das 2,5-Dihydrofuran zu 2,3-Dihydrofuran isomerisiert und anschließend aus der Reaktionsmischung abdestilliert wird. Sodann wird das 2,3-Dihydrofuran an einem aciden Katalysator mit Wasser zu einem Gemisch aus 4-Hydroxybutanal und 2-Hydroxytetrahydrofuran umgesetzt, welches in Gegenwart eines Hydrierkatalysators zu 1,4-Butandiol hydriert wird. Mithin handelt es sich hierbei um ein 3-stufiges Verfahren.

In EP-A 24 770 wird die einstufige Herstellung von 4-Hydroxybutanal durch die Umsetzung von 2,5-Dihydrofuran in Gegenwart großer Mengen Wasser und mittels eines Katalysators, der ein Metall der Gruppe VIIIb des Periodensystems der Elemente enthält und vor seiner Verwendung einer Behandlung mit einer Base unterzogen wurde, beschrieben. Dabei beträgt der Umsatz lediglich 55 % und die Selektivität ist mit 65 % ebenfalls relativ niedrig, so daß die Reaktionsmischung vor einer Hydrierung des 4-Hydroxybutyraldehyds zu 1,4-Butandiol zunächst destillativ aufgearbeitet werden mußte.

In US-A 4 859 801 (EP A 2 343 841) wird gelehrt, 2,3-Dihydrofuran in Gegenwart von Wasser bei einem pH-Wert von 8 bis 14 mit einem Aldehyd und Wasserstoff mittels eines Hydrierkatalysators zu Gemischen aus 1,4-Butandiol und 2-Alkyl-1,4-butandiol umzusetzen. Die Ausbeute an 1,4-Butandiol ist dabei bescheiden. Um 2,5-Dihydrofuran nach diesem Verfahren in 1,4-Butandiol umzuwandeln, müßte dieses in einer der beschriebenen Reaktion vorgelagerten Reaktionsstufe zunächst zu 2,3-Dihydrofuran isomerisiert werden.

BE-A 674 652 lehrt die Isomerisierung von 2,5-Dihydrofuran zu 2,3-Dihydrofuran mit Hilfe von Katalysatoren aus der Gruppe VIIIb des Periodensystems der Elemente.

Da nach all den genannten Verfahren eine mehrstufige Reaktionsführung erforderlich ist, um ausgehend von 2,5-Dihydrofuran zu 1,4-Butandiol zu gelangen, eine solche mehrstufige Verfahrensweise jedoch mit erheblichen Kosten verbunden ist, die den Preis des Verfahrensprodukts 1,4-Butandiol wettbewerbsunfähig verteuern, lag der vorliegenden Erfindung die Aufgabe zugrunde, ein Verfahren zu finden, das die Herstellung von 1,4-Butandiol in guten Ausbeuten und Selektivitäten ausgehend von 2,5-Dihydrofuran in einer Stufe ermöglicht.

Dementsprechend wurde ein Verfahren zur Herstellung von 1,4-Butandiol gefunden, das dadurch gekennzeichnet ist, daß man 2,5-Dihydrofuran einstufig, in Gegenwart von Wasser und Wasserstoff bei einer Temperatur von 20 bis 300°C und einem Druck von 1 bis 300 bar an einem Hydrierkatalysator umsetzt.

In dem erfindungsgemäßen Verfahren werden somit die 3 Reaktionsstufen a) Isomerisierung des 2,5-Dihydrofuran zu 2,3-Dihydrofuran gemäß Gleichung (1) 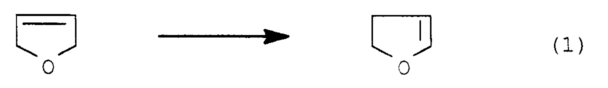 b) Umsetzung des 2,3-Dihydrofuran mit Wasser zu einem Gemisch aus 4-Hydroxybutyraldehyd und dessen Isomerem 2-Hydroxytetrahydrofuran gemäß Gleichung (2) 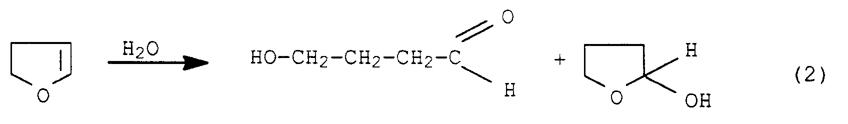 und c) die katalytische Hydrierung des gemäß Gleichung (2) erhaltenen Gemisches aus 4-Hydroxybutyraldehyd und 2-Hydroxytetrahydrofuran - beide Verbindungen stehen miteinander im Gleichgewicht - zu 1,4-Butandiol gemäß Gleichung (3) 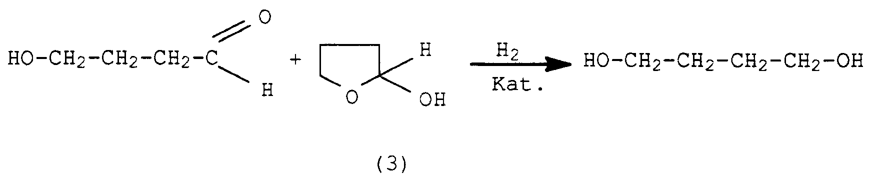 in einer einzigen Verfahrensstufe bewirkt.

Bei der Durchführung des erfindinngsgemäßen Verfahrens wird das 2,5-Dihydrofuran mit Wasser im allgemeinen in einem Molverhältnis 2,5-Dihydrofuran/Wasser von 1:1 bis 1:100, vorzugsweise von 1:1 bis 1:50 und besonders bevorzugt von 1:1 bis 1:10 und in Gegenwart von Wasserstoff und eines Hydrierkatalysators bei einem Druck von im allgemeinen 1 bis 300 bar, vorzugsweise von 5 bis 200 bar und insbesondere von im allgemeinen 10 bis 150 bar bei einer Temperatur von 20 bis 300°C, vorzugsweise von 40 bis 230°C und besonders bevorzugt von 80 bis 200°C zu 1,4-Butandiol umgesetzt.

Als Hydrierkatalysatoren können im erfindungsgemäßen Verfahren im allgemeinen alle zur Hydrierung von Carbonylgruppen geeigneten Katalysatoren Verwendung finden. Es können sowohl homogen im Reaktionsmedium lösliche Hydrierkatalysatoren, wie sie beispielsweise in Houben-Weyl, Methoden der Organischen Chemie, Band IV/1c, S. 45 bis 67, Thieme Verlag, Stuttgart 1980 beschrieben werden oder aber auch heterogene Hydrierkatalysatoren, wie sie in Houben-Weyl, Methoden der Organischen Chemie, Band IV/1c, S. 16 bis 26 beschrieben werden, benutzt werden. Bevorzugte Homogenkatalysatoren sind insbesondere die Komplexe des Rhodiums, Rutheniums und Kobalts mit Phosphin- oder Phosphit-Liganden, deren Herstellung z.B. in CA-A 7 276 41, H. Brunner in Hartley: The chemistry of the metal-carbon bond; Vol. 5, S. 110-124, John Wiley & Sons, New York 1989 sowie Tóth et al, Inorg. Chim. Acta 42, 153 (1980) und der dort zitierten Literatur beschrieben wird.

Vorzugsweise wird im erfindungsgemäßen Verfahren jedoch mit heterogenen Hydrierkatalysatoren gearbeitet, also solchen Hydrierkatalysatoren, die im Reaktionsmedium im wesentlichen unlöslich sind. Von diesen Hydrierkatalysatoren sind solche bevorzugt, die ein oder mehrere Elemente der Gruppe Ib, VIb, VIIb und VIIIb des Periodensystems der Elemente, insbesondere Kupfer, Chrom, Molybdän, Wolfram, Rhenium, Ruthenium, Kobalt, Rhodium, Iridium, Nickel, Palladium, Eisen und/oder Platin enthalten.

Im erfindungsgemäßen Verfahren können heterogene Hydrierkatalysatoren verwendet werden, die aus Metallen in aktivierter, feinverteilter Form mit großer Oberfläche bestehen, beispielsweise Raney-Nickel, Raney-Cobalt, Palladium-, Platin- oder Rhenium-Schwamm.

Des weiteren können im erfindungsgemäßen Verfahren z.B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder -Carbonat-Lösungen, als z.B. schwerlösliche Hydroxyde, Oxydhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die betreffenden Oxide, Mischoxide und/oder gemischtvalentigen Oxide umwandelt, welche durch eine Behandlung mit Wasserstoff oder mit Wasserstoff enthaltenden Gasen bei in der Regel 100 bis 700°C, insbesondere bei 150 bis 400°C, zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren, bei denen die katalytisch-hydrierend wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diese Trägermaterialien z.B. durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Hilfe von Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder mit thermisch leicht zersetzbaren Komplexverbindungen, z.B. mit Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfung oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen zu höheren Raum-Zeit-Umsätzen führen als niedrigere Gehalte. Im allgemeinen werden aber Trägerkatalysatoren verwendet, deren Gehalt an katalytisch aktiven Metallen 0,1 bis 80 Gew.-%, vorzugsweise 0,5 bis 30 Gew.-% und bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindundungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach den Verfahren von DE-A 25 19 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung auf einem abgeschiedenen Salze oder Komplexe der zuvor genannten Metalle, erzeugt werden.

Die Aktivierung der Fällungskatalysatoren als auch der Trägerkatalysatoren kann auch in situ in der Reaktionsmischung durch den dort anwesenden Wasserstoff erfolgen, bevorzugt werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Kieselgur, Kieselgel, Tonerden, z.B. Montmorillonite, Silikate, wie Magnesium- oder Aluminiumsilikate, Zeolithe, wie ZSM-5 oder ZSM-10-Zeolithe sowie Aktivkohle verwendet werden. Bevorzugte Trägermaterialien sind Aluminiumoxide, Titandioxide, Zirkoniumdioxid und Aktivkohle. Selbstverständlich können auch Mischungen verschiedener Trägermaterialien als Träger für im erfindungsgemäßen Verfahren anwendbare Katalysatoren dienen.

Als für im erfindungsgemäßen Verfahren einsetzbare Heterogenkatalysatoren seien die folgenden Katalysatoren beispielhaft genannt:
Platinschwarz, Platin auf Aktivkohle, Platindioxid, Palladium auf Aktivkohle, Palladium auf Aluminiumoxid, Palladium auf Siliciumdioxid, Palladium auf Bariumsulfat, Rhodium auf Aktivkohle, Rhodium auf Aluminiumoxid, Ruthenium auf Siliciumdioxid oder Aktivkohle, Nickel auf Siliciumdioxid, Raney-Nickel, Kobalt auf Siliciumdioxid, Kobalt auf Aluminiumoxid, Raney-Kobalt, Carbonyleisenpulver, Rheniumschwarz, Raney-Rhenium, Rhenium auf Aktivkohle, Rhenium-Palladium auf Aktivkohle, Rhenium-Platin auf Aktivkohle, Kupfer auf Siliciumdioxid, Kupfer auf Aluminiumoxid, Kupfer auf Aktivkohle, Raney-Kupfer, Platinoxid-Rhodiumoxid-Mischungen, Platin-Palladium auf Aktivkohle, Kupferchromit, Bariumchromit, Nickel-Chromoxid auf Aluminiumoxid, Dirheniumheptoxid (Re₂O₇), Kobaltsulfid, Nickelsulfid, Molybdän(VI)sulfid, Kupfer-Molybdän (VI) oxid-Siliciumdioxid-Aluminiumoxid-Katalysatoren sowie die Katalysatoren gemäß DE-A 39 32 332, US-A 3 449 445, EP-A 44 444, EP-A 147 219, DE-A 39 04 083, DE-A 23 21 101, EP-A 415 202, DE-A 2 366 264 und EP-A 100 406.

Den vorgenannten Katalysatoren können Lewis- und/oder Brönstedacide Komponenten, wie Zeolithe, Aluminium- oder Siliciumoxide, Phosphorsäure oder Schwefelsäure, zugesetzt werden. Sie werden im allgemeinen in Mengen von 0,01 bis 5 Gew.-%, vorzugsweise von 0,05 bis 0,5 Gew.-% und besonders bevorzugt von 0,1 bis 0,4 Gew.-%, bezogen auf das Gewicht des eingesetzten Katalysators.

Besonders bevorzugt wird das erfindungsgemäße Verfahren so ausgeführt, daß man Hydrierkatalysatoren verwendet, die Brönsted- und/oder Lewis-acide Zentren enthalten. Bei der Verwendung derartiger Katalysatoren ist im allgemeinen die zusätzliche Zugabe einer Brönsted- oder Lewis-Säure zur Reaktionsmischung nicht erforderlich.

Als Brönsted-Säurezentren-haltige Homogenkatalysatoren können z.B. Übergangsmetallkomplexe von Metallen der Gruppe VIIIb, insbesondere Rhodium-, Ruthenium- und Kobalt-Komplexe mit Phosphin- oder Phosphit-Liganden verwendet werden, welche Brönsted-acide funktionelle Gruppen, wie Carboxyl-, Sulfonsäure- und/oder Phosphonsäuregruppen als Substituenten tragen, beispielsweise Komplexe der genannten Übergangsmetalle mit Triphenylphosphin-p-sulfonsäure-Liganden. Solche Liganden können z.B. nach dem Verfahren von Angew. Chem. 105, 1097 (1993) hergestellt werden.

Besonders vorteilhafte Ergebnisse können im erfindungsgemäßen Verfahren mit Heterogenkatalysatoren, die Brönsted- oder Lewis-saure Zentren enthalten, erzielt werden. Als Brönsted- oder Lewis-saure Zentren können z.B. die katalytisch aktiven Metalle selber wirken, wenn diese bei der Aktivierung des Katalysators mit Wasserstoff oder Wasserstoff-enthaltenden Gase nicht vollständig zu den betreffenden Metallen reduziert werden. Dies gilt z.B. für die Rhenium- und Chromit-haltigen Katalysatoren wie Rheniumschwarz und Kuperchromit. Im Rheniumschwarz liegt das Rhenium als Mischung von Rheniummetall mit Rheniumverbindungen in höheren Oxidationsstufen vor, wobei letztere Wirkungen wie Lewis- oder Brönsted-Säuren entfalten können. Weiterhin können solche Lewis- oder Brönsted-aciden Zentren über das verwendete Trägermaterial in den Katalysator eingebracht werden. Als Lewis- oder Brönsted-acide Zentren enthaltende Trägermaterialien seien z.B. die Aluminiumoxide, Titandioxide, Zirkoniumdioxid, Siliciumdioxid, die Silikate, Tonerden, Zeolithe und Aktivkohle genannt.

Besonders bevorzugt werden deshalb im erfindungsgemäßen Verfahren als Hydrierkatalysatoren Trägerkatalysatoren verwendet, die Elemente der I., VI., VII. und/oder VIII. Nebengruppe des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Rhenium auf Aktivkohle, Rhenium auf Zirkoniumdioxid, Rhenium auf Titandioxid, Rhenium auf Siliciumdioxid, Kupfer auf Aktivkohle, Kupfer auf Siliciumdioxid und Ruthenium auf Aktivkohle.

Das erfindungsgemäße Verfahren kann sowohl kontinuierlich als auch diskontinuierlich ausgeübt werden. Zur kontinuierlichen Betriebsweise können beispielsweise Rohrreaktoren vorteilhaft eingesetzt werden, in denen der Katalysator vorteilhaft in Form eines Festbetts angeordnet ist, über das die Reaktionsmischung in der Sumpf- oder Rieselfahrweise geleitet werden kann. Bei der diskontinuierlichen Betriebsweise können sowohl einfache Rührreaktoren oder vorteilhaft Schlaufenreaktoren verwendet werden. Bei Verwendung von Schlaufenreaktoren wird der Katalysator zweckmäßigerweise in Form eines Festbetts angeordnet. Bei nichtvollständiger Umsetzung des Ausgangsmaterials kann dieses zweckmäßigerweise entweder nach destillativer Abtrennung der Wertprodukte oder als Teilstrom zusammen mit den anderen Reaktionsprodukten in die Umsetzung zurückgeführt werden. Dies kann sich insbesondere bei der kontinuierlichen Betriebsweise als vorteilhaft erweisen. Im allgemeinen werden bei kontinuierlicher Reaktionsführung höhere Ausbeuten an 1,4-Butandiol erhalten als bei diskontinuierlicher Reaktionsführung am selben Katalysator.

Das erfindungsgemäße Verfahren kann vorteilhaft in Anwesenheit eines unter den Reaktionsbedingungen inerten Lösungsmittels durchgeführt werden, beispielsweise mit wasserlöslichen Ethern, wie Tetrahydrofuran, Dioxan oder Dimethoxymethan. Vorteilhaft können auch Alkohole, insbesondere das Verfahrensprodukt 1,4-Butandiol, als Lösungsmittel eingesetzt werden.

Der Reaktionsaustrag des erfindungsgemäßen Verfahrens wird zweckmäßigerweise destillativ aufgearbeitet, wobei außer dem Verfahrensprodukt 1,4-Butandiol, die in geringen Mengen als Nebenprodukte anfallenden Verbindungen Tetrahydrofuran, γ-Butyrolacton und Butanol als Wertprodukte gewonnen werden können.

Das Ausgangsmaterial 2,5-Dihydrofuran kann durch die Isomerisierung von Vinyloxiran, beispielsweise nach dem Verfahren von US-A 5 034 545 oder US-A 5 082 956, hergestellt werden.

1,4-Butandiol wird weltweit in großem Umfang hergestellt und dient als Diolkomponente zur Herstellung von u.a. Polyestern, Polyurethanen und Epoxidharzen.

### Beispiele

### Beispiel 1

In einem 25 ml-fassenden Rohrreaktor wurden 25 ml eines zu 4 mm-Strängen geformten Kupfer auf Siliciumdioxid-Katalysators gefüllt, dessen Kupfergehalt, berechnet als Cu und bezogen auf den gesamten Katalysator 10 Gew.-% betrug. Der Katalysator war durch Tränken der Siliciumdioxid-Stränge mit einer ammoniakalischen Kupfercarbonat/Natriumnitrat-Lösung und anschließende Trocknung bei 100°C hergestellt worden. Der Katalysator wurde 2 Stunden lang bei 250°C in einer Wasserstoffatmosphäre (100 l H₂/h) aktiviert. Anschließend wurden 15 ml/h einer Mischung aus 2,8 Gew.-Teilen 2,5-Dihydrofuran, 2,8 Gew.-Teilen Wasser und 1 Gew.-Teil 1,4-Dioxan bei einem Wasserstoffdruck von 120 bar und einer Reaktortemperatur von 134°C kontinuierlich über den Katalysator geleitet.

Die Zusammensetzung des Reaktionsaustrages ist in der Tabelle angegeben.

### Beispiel 2

Analog Beispiel 1 wurde das dort beschriebene Gemisch aus 2,5-Dihydrofuran, Wasser und 1,4-Dioxan an einem Rhenium auf Aktivkohle-Katalysator, der einen Rhenium-Gehalt von 6 Gew.-%, berechnet als Re und bezogen auf das Gewicht des gesamten Katalysators, hatte, bei einem Wasserstoffdruck von 120 bar und einer Temperatur von 200°C umgesetzt. Der Rhenium auf Aktivkohle-Katalysator war durch Tränken der Aktivkohlestränge mit einer wäßrigen Dirheniumheptoxid-Lösung und anschließende Trocknung bei 120°C hergestellt worden. Vor seiner Verwendung wurde er 3 Stunden mit Wasserstoff bei 300°C aktiviert.

Die Zusammensetzung des Reaktionsaustrages ist in der Tabelle angegeben.

### Beispiel 3

Analog Beispiel 1 wurde ein Gemisch aus 2,5-Dihydrofuran (3,1 Gew.-Teile), 1,4-Dioxan (3,1 Gew.-Teile) und Wasser (1 Gew.-Teil) an einem Rhenium auf Titandioxid-Katalysator, der einen Rhenium-Gehalt von 6 Gew.-%, berechnet als Re und bezogen auf das Gewicht des gesamten Katalysators, hatte, bei einem Wasserstoffdruck von 120 bar und einer Temperatur von 154°C umgesetzt. Der Rhenium-Katalysator war vorher durch Tränken von Titandioxidsträngen mit wäßriger Rheniumheptoxid-Lösung und Trocknung bei 120°C hergestellt worden und wurde vor seiner Verwendung 3 Stunden im Wasserstoffstrom bei 300°C aktiviert.

Die Zusammensetzung des Reaktionsaustrags ist in der Tabelle angegeben.

### Beispiel 4

Analog Beispiel 1 wurde ein Gemisch aus 2,5-Dihydrofuran (1,5 Gew.-Teile) Tetrahydrofuran (1,1 Gew.-Teile) und Wasser (1 Gew.-Teil) am Rhenium auf Titandioxid-Katalysator gemäß Beispiel 3 bei 120 bar Wasserstoffdruck und einer Temperatur von 200°C umgesetzt.

In einem zweiten Versuch wurde die Zulaufmenge des Reaktionsgemisches auf 50 ml/h verdoppelt.

Die Zusammensetzungen der Reaktionsausträge sind in der Tabelle angegeben.

| Beispiel | Zulauf [ml/h] | BD | THF | GBL | HBA | BuOH | Umsatz [%] |
|---|---|---|---|---|---|---|---|
| | | [Mol-%] | | | | | |
| 1 | 25 | 32 | 15 | 0,3 | 1 | 2 | 51 |
| 2 | 25 | 38 | 4 | 9 | 5 | 4 | 61 |
| 3 | 20 | 85 | 6 | 3 | 1 | 5 | 100 |
| 4 | 25 | 79 | * | 5 | 0 | 9 | 100 |
| | 50 | 71 | * | 9 | 2 | 9 | 95 |
| BD: 1,4 Butandiol, THF: Tetrahydrofuran, GBL: γ-Butyrolacton; HBA: 4-Hydroxybutanal, BuOH: n-Butanol *: Das in den Versuchen neu gebildete THF wurde nicht bilanziert, da THF als Lösungsmittel verwendet wurde. Die Angabe Mol-% bezieht sich auf umgesetztes 2,5-Dihydrofuran. | | | | | | | |

### Beispiel 6

In einem 50 ml-Metallautoklaven mit Rührer wurden 2 g eines Kupfer auf Aktivkohle-Katalysators (Kupfergehalt: 10 Gew.-%, berechnet als Cu und bezogen auf das Gesamtgewicht des Katalysators), 5 g 2,5-Dihydrofuran und 2,6 g Wasser gefüllt. Der Katalysator war vorher durch Tränken der Aktivkohle mit einer wäßrigen Kupfernitratlösung, Trocknung bei 100°C und Aktivierung im Wasserstoffstrom während 2 h bei 250°C hergestellt worden. Durch Einpressen von Wasserstoff wurde im Autoklaven ein Druck von 50 bar erzeugt, sodann wurde der Autoklav für eine Stunde auf eine Temperatur von 150°C aufgeheizt. Danach wurde der Autoklav auf Raumtemperatur abgekühlt und entspannt. Bei einem Umsatz von 85 % wurden 31 Mol-% 1,4-Butandiol, 30 Mol-% Tetrahydrofuran, 2 Mol-% γ-Butyrolacton, 18 Mol-% 4-Hydroxybutyraldehyd und 1 Mol-% Butanol, jeweils bezogen auf die Menge des umgesetzten 2,5-Dihydrofuran, erhalten.

### Beispiel 7

Analog zu Beispiel 6 wurden 5 g 2,5-Dihydrofuran und 5,4 g Wasser an 2 g eines Ruthenium auf Aktivkohle-Katalysators umgesetzt, dessen Rutheniumgehalt, berechnet als Ru und bezogen auf den gesamten Katalysator, 3 Gew.-% betrug. Der Katalysator war durch Tränken der Aktivkohle mit wäßriger Rutheniumnitratlösung; Trocknen bei 100°C und Aktivierung im Wasserstoffatom während 2 h bei 250°C hergestellt worden. Bei einem 2,5-Dihydrofuran-Umsatz von 100 % wurden 51 Mol-% 1,4-Butandiol, 35 Mol-% Tetrahydrofuran, 1 Mol-% γ-Butyrolacton und 3 Mol-% Butanol, jeweils bezogen auf die Menge des umgesetzten 2,5-Dihydrofuran, erhalten.

### Beispiel 8

In einem 50 ml Metallautoklaven mit Rührer wurden 0,2 g Dirheniumheptoxid (Re₂O₇) in 5,1 g Wasser gelöst. Durch Einpressen von Wasserstoff wurde im Autoklaven ein Druck von 40 bar erzeugt, sodann wurde der Autoklav für 1 Stunde auf 300°C aufgeheizt. Nach Abkühlung und Entspannung des Autoklaven wurden 5 g 2,5-Dihydrofuran in den Reaktor gegeben. Es wurde erneut Wasserstoff bis zu einem Druck von 50 bar in den Autoklaven eingepreßt und anschließend der Autoklav für 1 Stunde auf 150°C aufgeheizt, sonach auf Raumtemperatur abgekühlt und entspannt. Bei einem 2,5-Dihydrofuranumsatz von 99 % wurden 45 Mol-% 1,4-Butandiol, 36 Mol-% Tetrahydrofuran, 1 Mol-% 4-Hydroxybutyraldehyd, 3 Mol-% γ-Butyrolacton und 12 Mol-% Butanol, jeweils bezogen auf die Menge des umgesetzten 2,5-Dihydrofuran, erhalten.

### Beispiel 9

In einem Metallautoklaven mit Rührer wurden 0,09 g des Homogenkatalysators RuHClCo(PPh₃)₃ (Ph bedeutet Phenyl), 5 g 2,5-Dihydrofuran und 5,1 g Wasser gefüllt und durch Einpressen von Wasserstoff ein Druck von 50 bar erzeugt. Sodann wurde der Autoklav 1 h auf 150°C aufgeheizt und anschließend auf Raumtemperatur abgekühlt und entspannt. Der homogene Reaktionsaustrag enthielt bei einem 2,5-Dihydrofuranumsatz von 90 %, 40 Mol-% 1,4-Butandiol, 10 Mol-% Tetrahydrofuran und 5 Mol-% Butanol, jeweils bezogen auf die Menge des umgesetzten 2,5-Dihydrofuran.

### Beispiel 10

In einen 25 ml fassenden Rohrreaktor wurden 25 ml eines Kupfer und Rhenium enthaltenden Aktivkohlekatalysators, der einen Kupfer-Gehalt von 3 Gew.-% Kupfer, berechnet als Kupfer, und einen Rhenium-Gehalt von 6 Gew.-% Rhenium, berechnet als Rhenium, jeweils bezogen auf das Gewicht des gesamten Katalysators, gefüllt. Der Katalysator war durch Tränken von 4 mm Aktivkohlesträngen mit einer wäßrigen Lösung von Kupfernitrat und Rheniumheptoxid, anschließender Trocknung bei 120°C und dreistündiger Aktivierung im Wasserstoffstrom bei 300°C hergestellt worden. Der Reaktor wurde in Rieselfahrweise bei 190°C und 120 bar wasserstoffdruck kontinuierlich betrieben. Es wurden 13 g 2,5-Dihydrofuran/h und 7 g Wasser/h über zwei getrennte Zulaufleitungen auf den Reaktorkopf gepumpt. Das Reaktorabgas betrug 50 l/h. Bei einem Umsatz von 98 % betrug die 1,4-Butandiolausbeute 69 %, die THF-Ausbeute 20 %, die Butyrolactonausbeute 4 % und die n-Butanolausbeuten 6 %. Der Rest war vorwiegend 4-Hydroxybutyraldehyd.

### Beispiel 11

In einen 400 ml Rohrreaktor wurden 400 ml des Katalysators aus Beispiel 2 gefüllt. Bei 190 - 200°C und 120 bar Wasserstoffdruck wurden 200 g 2,5-Dihydrofuran/h und 100 g Wasser/h über zwei getrennte Zulaufleitungen auf den Kopf des Reaktors gepumpt. Der Reaktor wurde mit Produktrückführung betrieben, wobei das Umlauf: Zulauf-Verhältnis 10 : 1 betrug. Der Abgasstrom betrug 100 l/h. Der Reaktor wurde 30 Tage kontinuierlich betrieben und während dieser Zeit Proben entnommen, wobei bei 100 % Umsatz folgende Ausbeuten erhalten wurden: 1,4-Butandiol: 65 - 69 %,
THF: 25 - 30 %, Butyrolacton: 1 - 3 %, n-Butanol 5 - 6 %. Nach 30 Tagen war noch keine Katalysatordesaktivierung eingetreten.

## Patentansprüche

1. Verfahren zur Herstellung von 1,4-Butandiol, dadurch gekennzeichnet, daß man 2,5-Dihydrofuran einstufig, in Gegenwart von Wasser und Wasserstoff bei einer Temperatur von 20 bis 300°C und einem Druck von 1 bis 300 bar an einem Hydrierkatalysator umsetzt.

2. Verfahren nach Anspruch 1, dadurch gekennzeichnet, daß man einen Hydrierkatalysator verwendet, der mindestens ein Element aus der Gruppe Ib, VIb, VIIb oder VIIIb des Periodensystems der Elemente enthält.

3. Verfahren nach den Ansprüchen 1 und 2, dadurch gekennzeichnet, daß man einen heterogenen Hydrierkatalysator einsetzt.

4. Verfahren nach den Ansprüchen 1 bis 3, dadurch gekennzeichnet, daß man einen Hydrierkatalysator verwendet, dessen katalytisch aktive Komponente auf einen Träger aufgebracht worden ist.

5. Verfahren nach den Ansprüchen 1 bis 4, dadurch gekennzeichnet, daß man einen Hydrierkatalysator verwendet, der eine oder mehrere Brönsted- oder Lewis-acide wirkende Komponenten enthält.

6. Verfahren nach den Ansprüchen 1 bis 5, dadurch gekennzeichnet, daß man einen Hydrierkatalysator verwendet, der Rhenium enthält.

7. Verfahren nach den Ansprüchen 1 bis 5, dadurch gekennzeichnet, daß man einen Hydrierkatalysator verwendet, der Kupfer enthält.

8. Verfahren nach den Ansprüchen 1 bis 5, dadurch gekennzeichnet, daß man einen Hydrierkatalysator verwendet, der Nickel oder Kobalt enthält.

9. Verfahren nach den Ansprüchen 1 bis 5, dadurch gekennzeichnet, daß man einen Hydrierkatalysator verwendet, der ein Platinmetall enthält.

10. Verfahren nach den Ansprüchen 1 bis 9, dadurch gekennzeichnet, daß man einen Hydrierkatalysator verwendet, dessen katalytisch aktive Komponente auf einem Trägermaterial aufgebracht worden ist, das Aluminiumoxid, Tonerden, Siliciumdioxid, Zirkoniumdioxid, Titandioxid, einen Zeolithen und/oder Aktivkohle enthält.

11. Verfahren nach den Ansprüchen 1 und 2, dadurch gekennzeichnet, daß man einen homogenen Hydrierkatalysator verwendet, der ein Element der Gruppe VIIIb des Periodensystems der Elemente enthält.

## Claims

1. A process for preparing 1,4-butanediol, which comprises reacting 2,5-dihydrofuran in one stage in the presence of water and hydrogen at from 20 to 300°C and under a pressure of from 1 to 300 bar on a hydrogenation catalyst.

2. A process as claimed in claim 1, wherein a hydrogenation catalyst which contains at least one element from group Ib, VIb, VIIb or VIIIb of the periodic table of the elements is used.

3. A process as claimed in claim 1 or 2, wherein a heterogeneous hydrogenation catalyst is employed.

4. A process as claimed in any of claims 1 to 3, wherein a hydrogenation catalyst whose catalytically active component has been applied to a carrier is used.

5. A process as claimed in any of claims 1 to 4, wherein a hydrogenation catalyst which contains one or more components with Brönsted or Lewis acid activity is used.

6. A process as claimed in any of claims 1 to 5, wherein a hydrogenation catalyst which contains rhenium is used.

7. A process as claimed in any of claims 1 to 5, wherein a hydrogenation catalyst which contains copper is used.

8. A process as claimed in any of claims 1 to 5, wherein a hydrogenation catalyst which contains nickel or cobalt is used.

9. A process as claimed in any of claims 1 to 5, wherein a hydrogenation catalyst which contains a platinum metal is used.

10. A process as claimed in any of claims 1 to 9, wherein a hydrogenation catalyst whose catalytically active component has been applied to a carrier material which contains aluminum oxide, aluminas, silicon dioxide, zirconium dioxide, titanium dioxide, a zeolite and/or active carbon is used.

11. A process as claimed in claim 1 or 2, wherein a homogeneous hydrogenation catalyst which contains an element of group VIIIb of the periodic table of the elements is used.

## Revendications

1. Procédé de préparation de 1,4-butanediol caractérisé en ce que l'on fait réagir en une étape du 2,5-dihydrofuranne sur un catalyseur d'hydrogénation, en présence d'eau et d'hydrogène à une température de 20 à 300°C et sous une pression de 1 à 300 bar.

2. Procédé selon la revendication 1, caractérisé en ce que l'on utilise un catalyseur d'hydrogénation qui contient au moins un élément des groupes Ib, VIb, VIIb ou VIII de la classification périodique des éléments.

3. Procédé selon les revendications 1 ou 2, caractérisé en ce que l'on utilise un catalyseur d'hydrogénation hétérogène.

4. Procédé selon l'une quelconque des revendications 1 à 3, caractérisé en ce que l'on utilise un catalyseur d'hydrogénation dont le composant catalytique actif a été déposé sur un support.

5. Procédé selon l'une quelconque des revendications 1 à 4, caractérisé en ce que l'on utilise un catalyseur d'hydrogénation qui contient un ou plusieurs composants à action acide au sens de Lewis ou de Brönsted.

6. Procédé selon l'une quelconque des revendications 1 à 5, caractérisé en ce que l'on utilise un catalyseur d'hydrogénation contenant du rhénium.

7. Procédé selon l'une quelconque des revendications 1 à 5, caractérisé en ce que l'on utilise un catalyseur d'hydrogénation contenant du cuivre.

8. Procédé selon l'une quelconque des revendications 1 à 5, caractérisé en ce que l'on utilise un catalyseur d'hydrogénation contenant du nickel ou du cobalt.

9. Procédé selon l'une quelconque des revendications 1 à 5, caractérisé en ce que l'on utilise un catalyseur d'hydrogénation contenant un métal du groupe du platine.

10. Procédé selon l'une quelconque des revendications 1 à 9, caractérisé en ce que l'on utilise un catalyseur d'hydrogénation, dont le composant catalytique actif a été déposé sur un support, contenant de l'oxyde d'aluminium, de l'alumine, du dioxyde de silicium, du dioxyde de zirconium, du dioxyde de titane, un zéolithe et/ou du charbon actif.

11. Procédé selon les revendications 1 ou 2, caractérisé en ce que l'on utilise un catalyseur d'hydrogénation homogène contenant un élément du groupe VIII de la classification périodique des éléments.
